# EUROPEAN PATENT APPLICATION

(11) **EP 4 245 323 A1**
(43) Date of publication of application: **20.09.2023**
(21) Application number: 21891410.9
(22) Date of filing: 23.06.2021
(51) Int. Cl.: A61L 2/10, A61L 9/20

(54) **STERILIZATION SYSTEM, STERILIZATION DEVICE, CONTROL DEVICE, CONTROL METHOD, AND CONTROL PROGRAM**

(30) Priority: 16.11.2020 JP 2020190086
(71) Applicant: Minebea Mitsumi Inc., Kitasaku-gun, Nagano 3890293 (JP)
(72) Inventor: CHIBA, Naohiro, Kitasaku-gun, Nagano 389-0293 (JP)
(74) Representative: Zabel, Julia Elisabeth
(86) International application number: PCT/JP2021/023723
(87) International publication number: WO 2022/102162

(57) **Abstract**

The sterilization system according to an embodiment comprises a light source unit (21), a horizontal angle adjustment unit (4) and/or vertical angle adjustment unit (3), a first sensor unit (61), a second sensor unit (62), and a control device (5). The light source unit (21) radially emits ultraviolet rays. The horizontal angle adjustment unit (4) adjusts the horizontal angle of the light source unit (21) by rotating the light source unit (21) in the horizontal direction. The vertical angle adjustment unit (3) adjusts the vertical angle of the light source unit (21) by rotating the light source unit (21) in the vertical direction. The first sensor unit (61) senses an object having a body temperature. The second sensor unit (62) senses the proximity of the object. The control device (5) reads setting information including the horizontal angle and/or the vertical angle, controls the rotation of the horizontal angle adjustment unit (4) and/or the vertical angle adjustment unit (3), and controls the emission of ultraviolet rays by the light source unit (21) according to the sensing states of the first sensor unit (61) and the second sensor unit (62).

## Description

### Technical Field

The present invention relates to a sterilization system, a sterilizer, a controller, a control method, and a control program.

### Background Art

Sterilization or decontamination through ultraviolet ray irradiation has been performed as a known practice (see, for example, Patent Documents 1 to 4 and the like).

### Citation List

### Patent Literature

Patent Document 1: Japanese Unexamined Patent Application Publication (Translation of PCT Application) No. 2017-528258 A
Patent Document 2: JP 2018-130535 A
Patent Document 3: JP 2018-69028 A
Patent Document 4: JP 5197550 B

### Summary of Invention

### Technical Problem

However, since ultraviolet rays largely affect a human body or the like and are harmful to the human body or the like, safety is required to be guaranteed for ultraviolet ray irradiation in a space where a person enters and leaves. Although there are ultraviolet rays with a wavelength confirmed to be safe to the human body, the time and distance for enabling irradiation to be performed has been defined, and irradiation falling outside the definition is harmful.

An object of the present invention made in view of the above is to provide a sterilization system, a sterilizer, a controller, a control method, and a control program capable of improving safety for a human body or the like.

### Solution to Problem

A sterilizer according to an aspect of the present invention to solve the problem described above and achieve the object includes a light source unit, a horizontal angle adjustment unit and/or a vertical angle adjustment unit, a first sensor unit, a second sensor unit, and a controller. The light source unit emits ultraviolet rays in a radiating form. The horizontal angle adjustment unit adjusts a horizontal angle of the light source unit by causing rotation of the light source unit in a horizontal direction. The vertical angle adjustment unit adjusts a vertical angle of the light source unit by causing rotation of the light source unit in a vertical direction. The first sensor unit detects an object having a body temperature. The second sensor unit detects proximity of an object. The controller reads setting information including the horizontal angle and/or the vertical angle, controls the rotation caused by the horizontal angle adjustment unit and/or the vertical angle adjustment unit, and controls irradiation of the ultraviolet rays by the light source unit based on a status of detection by the first sensor unit and the second sensor unit.

The sterilization system according to one aspect of the present invention can improve safety for a human body or the like.

### Brief Description of Drawings

FIG. 1 is a block diagram illustrating an example of a configuration of a sterilizer and the like according to an embodiment.
FIG. 2 is a diagram illustrating an example of a configuration in a case where a plurality of sterilizers are included.
FIG. 3 is a perspective view illustrating an example of a mechanical configuration of an example of the sterilizer.
FIG. 4 is a perspective view illustrating another example of a mechanical configuration of an example of the sterilizer.
FIG. 5 is a diagram illustrating an example of a hardware configuration of a control unit of a controller, a control unit of a terminal, or a control unit of a controller.
FIG. 6 is a diagram illustrating an example of a functional configuration of the control unit of the controller, the control unit of the terminal, or the control unit of the controller.
FIG. 7 is a diagram illustrating an example of a data structure of setting information held at the control unit of the controller, the control unit of the terminal, or the control unit of the controller.
FIG. 8 is a flowchart illustrating an example of control by the control unit of the controller, the control unit of the terminal, or the control unit of the controller.
FIG. 9 is a flowchart illustrating an example of processing in step S5 in FIG. 8.
FIG. 10 is a plan view illustrating an example of an area being an ultraviolet ray irradiation target.
FIG. 11 is a flowchart illustrating another example of the processing in step S5 in FIG. 8.

### Description of Embodiments

A sterilization system, a sterilizer, a controller, a control method, and a control program according to an embodiment will be described below with reference to the drawings. Note that the present invention is not limited to the embodiment described above. Furthermore, the dimensional relationships between elements, proportions of the elements, and the like in the drawings may differ from reality. The drawings may each include parts having mutually different dimensional relationships and proportions. Furthermore, the contents described in one embodiment or modified example are applied in principle to other embodiments or modification examples.

### Device Configuration, System Configuration

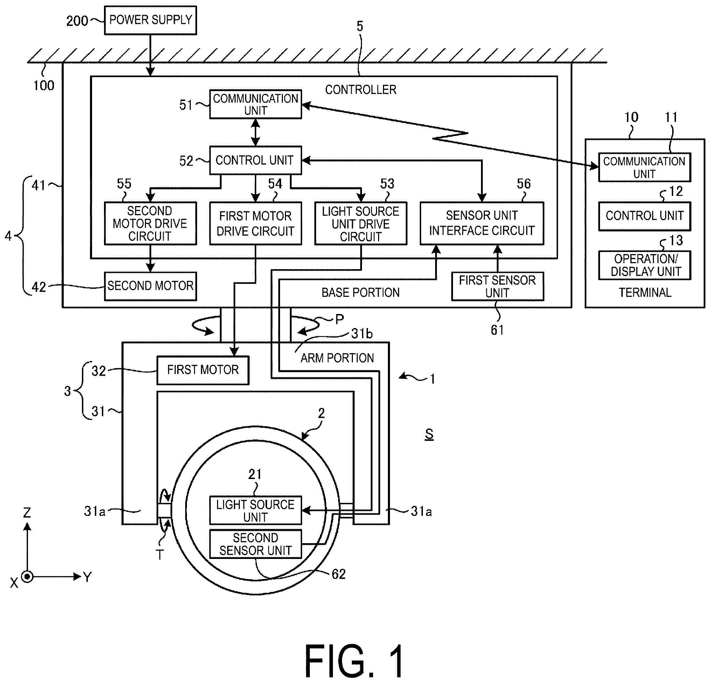

FIG. 1 is a block diagram illustrating an example of a configuration of a sterilizer 1 and the like according to an embodiment. In FIG. 1, the sterilizer 1 has a base portion 41 attached to a ceiling wall 100 forming a ceiling, for example, and includes: a main body portion 2 having a light source unit 21; a vertical angle adjustment unit 3 and a horizontal angle adjustment unit 4 rotatably supporting the main body portion 2; and a controller 5.

An X direction in FIG. 1 is a horizontal direction and is a front and back direction of the sterilizer 1. A Y direction is a horizontal direction and is a width direction of the sterilizer 1. A Z direction is a vertical direction orthogonal to the horizontal direction and is an up and down direction of the sterilizer 1. In the present embodiment, the Z direction is parallel to the vertical direction of a sterilization target space S, but is not limited to the vertical direction, and the Z direction may be inclined with respect to the vertical direction or the Z direction may be parallel to a direction orthogonal to the vertical direction.

The controller 5 is a portion performing control in the sterilizer 1. The controller 5 controls the light source unit 21, the vertical angle adjustment unit 3, and the horizontal angle adjustment unit 4, and is contained in an internal space of the base portion 41. The controller 5 includes a communication unit 51, a control unit 52, a light source unit drive circuit 53, a first motor drive circuit 54, a second motor drive circuit 55, and a sensor unit interface circuit 56. Based on electric power supplied from a power supply 200, the controller 5 performs turning ON/OFF of emission of ultraviolet rays by the light source unit 21, adjustment of the irradiation intensity, adjustment of a vertical angle of the light source unit 21 by the vertical angle adjustment unit 3, adjustment of a horizontal angle of the light source unit 21 by the horizontal angle adjustment unit 4, and the like. The hardware configuration of the controller 5 is the same as the hardware configuration of a general controller, and will be described in detail below. The power supply 200 is a commercial power supply, a generator, a battery, or the like.

The communication unit 51 transmits and receives information to and from the terminal 10 carried by a user, and, for example, receives operation information from the terminal 10, and transmits a status of the sterilizer 1, an irradiation status (for example, ON/OFF, irradiation intensity) of the light source unit 21, and an orientation status (for example, the vertical angle and the horizontal angle) of the light source unit 21 to the terminal 10. The communication unit 51 performs short-range wireless communications using Wi-Fi (registered trademark), Bluetooth (registered trademark), or the like for example.

The control unit 52 performs irradiation control on the light source unit 21, drive control on a first motor 32, drive control on a second motor 42, and the like. Here, the irradiation control includes ON/OFF control and irradiation intensity control on the light source unit 21.

The light source unit drive circuit 53 serves an interface between the light source unit 21 and the control unit 52, and supplies electric power to the light source unit 21 based on an irradiation control signal for the light source unit 21 from the control unit 52. When the light source unit 21 is of a type capable of changing the irradiation intensity of ultraviolet rays, the light source unit drive circuit 53 also adjusts the irradiation intensity. In addition, when the light source unit drive circuit 53 is of a type capable of changing (switching) the wavelength of the ultraviolet rays emitted by the light source unit 21, the light source unit drive circuit 53 also changes (switches) the wavelength. The light source unit drive circuit 53 is electrically connected to the control unit 52 and the light source unit 21. The light source unit drive circuit 53 is included in the controller 5, but is not limited to being included in the controller 5, and may be included in the light source unit 21. When the light source unit 21 is a light source combined with light sources of different wavelength ranges, adjustment is performed so that the irradiation intensity of each of the light sources can be adjusted.

The first motor drive circuit 54, serves as an interface between the first motor 32 and the control unit 52, and supplies driving electric power to the first motor 32 based on a drive control signal for the first motor 32 from the control unit 52, to rotate the light source unit 21 in the vertical direction. The first motor drive circuit 54 is electrically connected to the control unit 52 and the first motor 32. The first motor drive circuit 54 is included in the controller 5 but is not limited to being included in the controller 5, and may be included in the first motor 32.

The second motor drive circuit 55, serves as an interface between the second motor 42 and the control unit 52, and supplies driving electric power to the second motor 42 based on a drive control signal for the second motor 42 from the control unit 52, to rotate the light source unit 21 in the horizontal direction. The second motor drive circuit 55 is electrically connected to the control unit 52 and the second motor 42. The second motor drive circuit 55 is included in the controller 5 but is not limited to being included in the controller 5, and may be included in the second motor 42.

The sensor unit interface circuit 56 serves as an interface between a first sensor unit 61 provided on the base portion 41 side and a second sensor unit 62 provided on the light source unit 21 side, and transmits detection signals of the first sensor unit 61 and the second sensor unit 62 to the control unit 52. The first sensor unit 61 is a pyroelectric infrared sensor detecting an object having a body temperature within a predetermined distance (for example, within 7 m), and is disposed at a fixed position on the base portion 41 side to overlook the entire range to be irradiated with ultraviolet rays from the light source unit 21. The first sensor unit 61 may be disposed at a position different from the position of the base portion 41 as long as the first sensor unit 61 can detect an object in an area being the ultraviolet ray irradiation target. The first sensor unit 61 can be separated from the sterilizer 1 and provided in the proximity of the sterilizer 1, as long as the first sensor unit 61 is disposed at a position where the object in the area being the ultraviolet ray irradiation target can be detected. In this case, the first sensor unit 61, being a separate body, transmits a detection signal to the control unit 52 via wired or wireless communication with the sterilizer 1. The second sensor unit 62 is a proximity sensor detecting proximity (for example, proximity within 30 cm) of an object (not limited to an object having a body temperature), and is disposed at a movable position to be oriented in the irradiation direction of the ultraviolet rays together with the light source unit 21.

The user operates the terminal 10 to operate the sterilizer 1, and to recognize the status of the sterilizer 1. The terminal 10 includes a communication unit 11, a control unit 12, and an operation/display unit 13. The terminal 10 may be a dedicated terminal for the sterilizer 1, or may be a general-purpose terminal such as a smartphone, tablet, and laptop computer. The hardware configuration of the terminal 10 is similar to the hardware configuration of a general terminal, and will be described in detail below.

The communication unit 11 transmits and receives information to and from the controller 5. The communication unit 11 performs short-range wireless communications using Wi-Fi (registered trademark), Bluetooth (registered trademark), or the like for example. Note that transmission and reception of information is not limited to wireless, and may be wired transmission and reception.

The control unit 12 controls the terminal 10 and outputs operation information to the communication unit 11 based on at least an operation on the operation/display unit 13 by the user. The control unit 12 also controls display by the operation/display unit 13. The control unit 12 is electrically connected to the communication unit 11 and the operation/display unit 13. Note that the control unit 12 can take over part of processing of the control unit 52 connected via the communication unit 11 on the terminal side and the communication unit 51 on the sterilizer 1 side.

The operation/display unit 13 is used for a remote operation of the sterilizer 1 in addition to a general operation and display in the terminal 10. Although not illustrated, the operation/display unit 13 includes, for example, a switch corresponding to turning ON/OFF of the ultraviolet rays, a switch corresponding to the irradiation intensity, a switch corresponding to adjustment of the vertical angle of the light source unit 21, and a switch corresponding to adjustment of the horizontal angle of the light source unit 21. Note that the switches may be a mechanical switch such as an energized/de-energized switch or a resistance change switch, or may be an electrical switch such as a touch panel.

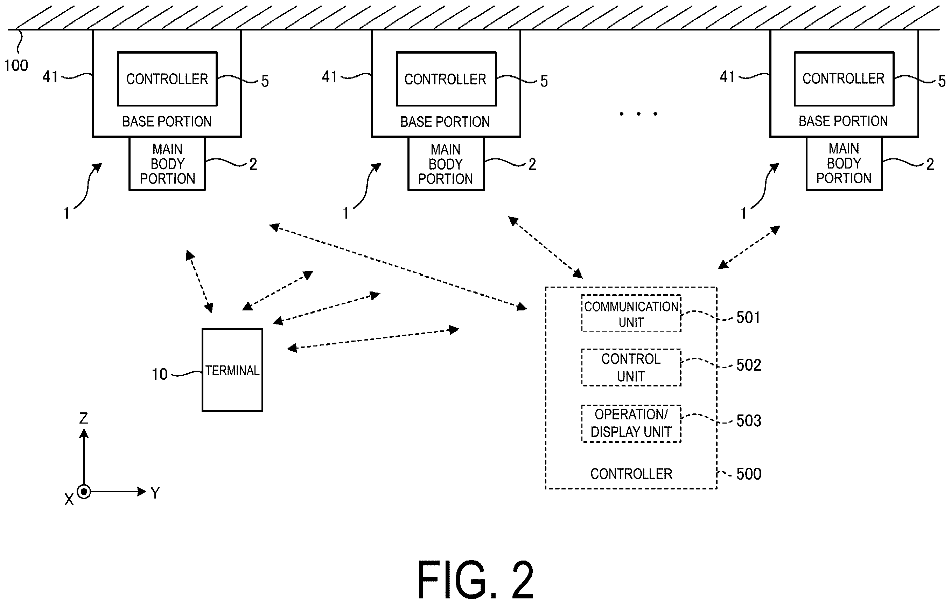

FIG. 2 is a diagram illustrating an example of a configuration in a case where a plurality of the sterilizers 1 are included. In FIG. 2, the plurality of sterilizers 1 are attached to the ceiling wall 100 forming a ceiling, for example. The controllers 5 of the respective sterilizers 1 perform control in an equal relationship, or one of the controllers 5 serves as a master to perform overall control. A controller 500 for the overall control may be provided separately from the controllers 5 of the sterilizers 1. The controller 500 includes a communication unit 501, a control unit 502, and an operation/display unit 503.

The communication unit 501 transmits and receives information to and from the controller 5 of each of the sterilizers 1. The communication unit 501 performs short-range wireless communications using Wi-Fi (registered trademark), Bluetooth (registered trademark), or the like for example. Note that transmission and reception of information is not limited to wireless, and may be wired transmission and reception.

The control unit 502 controls the controller 500 and outputs operation information to the communication unit 501 based on at least an operation on the operation/display unit 503 by the user or information set in advance. The control unit 502 is electrically connected to the communication unit 501 and the operation/display unit 503.

The operation/display unit 503 is used for a remote operation of each of the sterilizers 1 in addition to a general operation and display in the controller 500. Although not illustrated, the operation/display unit 503 includes, for example, a switch corresponding to turning ON/OFF of the ultraviolet rays, a switch corresponding to the irradiation intensity, a switch corresponding to adjustment of the vertical angle of the light source unit 21, a switch corresponding to adjustment of the horizontal angle of the light source unit 21, and a switch for setting various automatic operations. Note that the switches may be a mechanical switch such as an energized/deenergized switch or a resistance change switch, or may be an electrical switch such as a touch panel.

The terminal 10 communicates with the controller 5 of each of the sterilizers 1, with the controller 5 set to be the master, or with the controller 500, to correspond to various operations and status confirmation by the user. The terminal 10 can control each sterilizer 1 together with the controller 500 or in place of the controller 500.

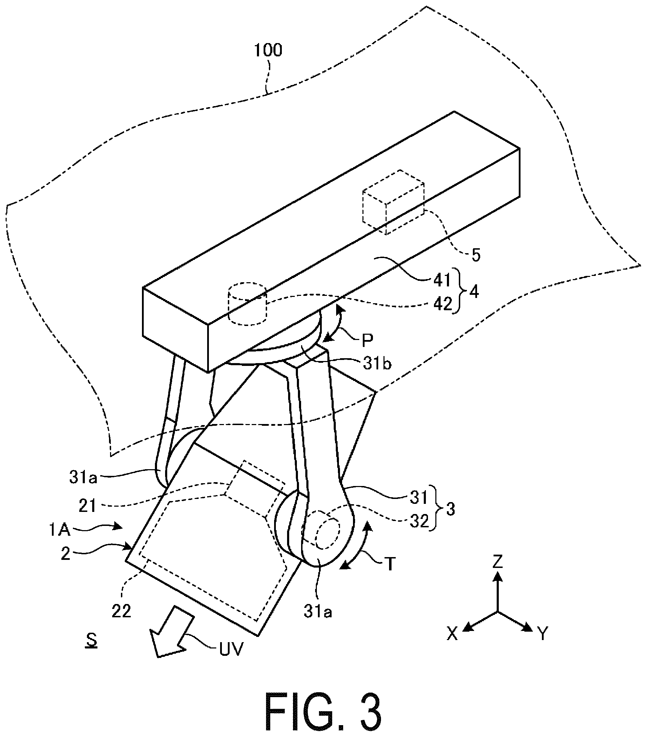

FIG. 3 is a perspective view illustrating an example of a mechanical configuration of an example of the sterilizer 1 (sterilizer 1A). As illustrated in FIG. 3, the sterilizer 1A irradiates the sterilization target space S with ultraviolet rays UV. The sterilizer 1A is attached to a wall forming a part of the sterilization target space S, that is, the ceiling wall 100 forming the ceiling in the present embodiment. Specifically, the sterilizer 1A is attached to the ceiling wall 100 via a rail or the like not illustrated, in a state of protruding from the ceiling wall 100 into the sterilization target space S. The sterilizer 1A includes the main body portion 2 with the light source unit 21 attached, the vertical angle adjustment unit 3, the horizontal angle adjustment unit 4, and the controller 5.

The light source unit 21 is attached to the main body portion 2. The main body portion 2 is formed in a substantially cylindrical shape, with an opening 22 formed at the sterilization target space S side in an axial direction and with the side opposite to the sterilization target space S closed. The main body portion 2 is supported, via a supporting shaft not illustrated, to be rotatable with respect to the vertical angle adjustment unit 3.

The light source unit 21 irradiates the sterilization target space S with the ultraviolet rays UV in a radiating form. The light source unit 21 is attached to a position opposing the opening 22 in the axial direction of the main body portion 2, in the internal space of the main body portion 2. The light source unit 21 irradiates the sterilization target space S with the ultraviolet rays UV through the opening 22. The ultraviolet rays UV to be emitted to the sterilization target space S by the light source unit 21 are electromagnetic waves having wavelengths different from the wavelength of visible light. The ultraviolet rays UV in a radiating form may be emitted from the light source, or the ultraviolet rays of a radiating form may be obtained by a lens not illustrated from the ultraviolet rays UV in a linear form emitted from the light source.

The light source unit 21 is not limited to a light source emitting the ultraviolet rays UV in one wavelength range, and may be capable of switching between ultraviolet rays UV in different wavelength ranges. The light source unit 21 may be a combination of the light source emitting the ultraviolet rays UV, and a light source emitting electromagnetic waves, for example, visible light or infrared rays in a wavelength range different from the wavelength range of the ultraviolet rays UV. The light source unit 21 may also be a combination of a light source emitting ultraviolet rays in one wavelength range, and a light source emitting ultraviolet rays in a wavelength range different from the wavelength range.

The vertical angle adjustment unit 3 rotates the light source unit 21 in the vertical direction Z, to adjust the vertical angle, that is, a tilt direction T. The vertical angle adjustment unit 3 includes an arm portion 31 and a first motor 32. The main body portion 2 is supported by the arm portion 31 to be rotatable in the vertical direction Z. The arm portion 31 is formed in a U-shape opening in the downward direction, and the main body portion 2 is supported, at both distal end portions 31a and 31a forming the opening, to be rotatable about a direction, and in the direction, the both distal end portions 31a and 31a oppose each other. The first motor 32 is a vertical actuator, and rotates the main body portion 2 with respect to the arm portion 31. The first motor 32 is attached to the arm portion 31 and rotates the main body portion 2 about the supporting shaft, that is, about the opposing direction, via a driving mechanism not illustrated. The light source unit 21 of the present embodiment is swung by the first motor 32 in a range of, for example, ±90 degrees when the downward direction is defined as 0 degrees. The vertical angle adjustment unit 3 is supported, via a supporting shaft not illustrated, to be rotatable with respect to the horizontal angle adjustment unit 4.

The horizontal angle adjustment unit 4 adjusts a horizontal angle, that is, adjusts a pan direction P by causing rotation of the light source unit 21 in the horizontal direction (the front and back direction X and the width direction Y). The horizontal angle adjustment unit 4 includes the base portion 41 and the second motor 42. The main body portion 2 is supported by the base portion 41 to be rotatable in the horizontal direction, via the arm portion 31. The base portion 41 is attached to the ceiling wall 100 and formed in a cubic shape with a longitudinal direction being in the front and back direction. On the base portion 41, a distal end portion 31b of the arm portion 31 in an upper direction side is supported so that the arm portion 31 is supported to be rotatable in the vertical direction Z. The second motor 42 is a horizontal actuator and rotates the arm portion 31 with respect to the base portion 41. The second motor 42 is attached to the base portion 41, and rotates the arm portion 31 about the supporting shaft, that is, the vertical direction, via a driving mechanism not illustrated, to rotate the main body portion 2. The light source unit 21 of the present embodiment is swung by the second motor 42 in a range of, for example, ±180 degrees when any of the horizontal directions is defined as 0 degrees.

In the above description, each actuator is a motor, but the motor is not limited to the actuator, and may be a hydraulic cylinder, a pneumatic cylinder, or the like.

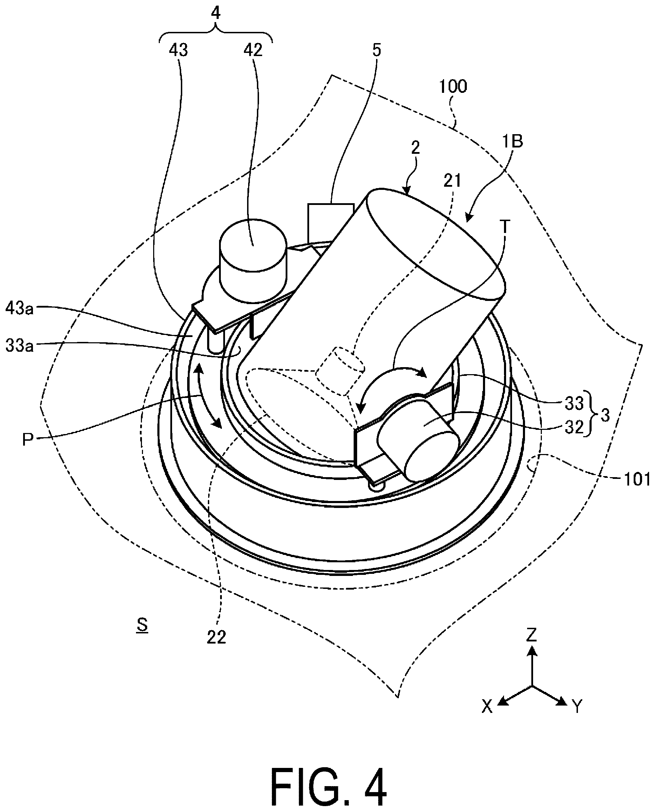

FIG. 4 is a perspective view illustrating an example of a mechanical configuration of another example of the sterilizer 1 (sterilizer 1B). In FIG. 4, the sterilizer 1B differs from the above-described sterilizer 1A in the state of attachment to the ceiling wall 100. Since the basic configuration of the sterilizer 1B is identical or substantially identical to the basic configuration of the sterilizer 1A, description of identical reference numerals will be omitted or simplified.

As illustrated in FIG. 4, the sterilizer 1B irradiates the sterilization target space S with ultraviolet rays UV. The sterilizer 1B is attached to the ceiling wall 100 forming a part of the sterilization target space S. Specifically, the sterilizer 1A is attached to the ceiling wall 100 while protruding from the ceiling wall 100 toward the side opposite to the sterilization target space S side, that is, while being buried in the ceiling wall 100. An opening 101 is formed at the ceiling wall 100, and the sterilizer 1B is attached with the sterilizer 1B, in particular, the opening 22 of the main body portion 2 exposed to the sterilization target space S through the opening 101. The sterilizer 1B includes the main body portion 2 with the light source unit 21 attached, the vertical angle adjustment unit 3, the horizontal angle adjustment unit 4, and the controller 5.

The light source unit 21 irradiating the sterilization target space S with the ultraviolet rays UV in a radiating form is attached to the main body portion 2. The main body portion 2 is formed in a substantially cylindrical shape, with an opening 22 formed at the sterilization target space S side in the axial direction and with the side opposite to the sterilization target space S side closed. The main body portion 2 is supported, via a supporting shaft not illustrated, to be rotatable with respect to the vertical angle adjustment unit 3.

The vertical angle adjustment unit 3 rotates the light source unit 21 in the vertical direction Z, to adjust the vertical angle, that is, a tilt direction T. The vertical angle adjustment unit 3 includes a sub frame portion 33 and the first motor 32. The main body portion 2 is supported by the sub frame portion 33 to be rotatable in the vertical direction Z. The sub frame portion 33, having an internal space 33a formed communicating with the outside through an opening in the upward and downward direction, is formed in a cylindrical shape with the axis in the up and down direction. In the internal space 33a of the sub frame portion 33, the main body portion 2 is supported to be rotatable about the horizontal direction. Specifically, in the sub frame portion 33, at least part of the main body portion 2 is accommodated in the internal space 33a, and the main body portion 2 is supported to be rotatable about the vertical direction. The first motor 32 is attached to the sub frame portion 33 and rotates the main body portion 2 about the supporting shaft, that is, about the opposing direction, via a driving mechanism not illustrated. The light source unit 21 of the present embodiment is swung by the first motor 32 in a range of, for example, -35 degrees to +45 degrees when the downward direction is defined as 0 degrees.

The horizontal angle adjustment unit 4 adjusts a horizontal angle, that is, adjusts a pan direction P by causing rotation of the light source unit 21 in the horizontal direction (the front and back direction X and the width direction Y). The horizontal angle adjustment unit 4 includes a base frame portion 43 and the second motor 42. The main body portion 2 is supported by the base frame portion 43 to be rotatable in the horizontal direction, via the sub frame portion 33. The base frame portion 43, with an internal space 43a formed communicating with the outside through an opening in the upward and downward direction, is attached to the ceiling wall 100 while opposing the opening 101 in the upward and downward direction, and is formed in a cylindrical shape with the axis in the upward and downward direction. In the internal space 43a of the base frame portion 43, the main body portion 2 is supported to be rotatable about the vertical direction. Specifically, in the base frame portion 43, at least part of the sub frame portion 33 is accommodated in the internal space 43a, and the sub frame portion 33 is supported to be rotatable about the vertical direction. The light source unit 21 of the present embodiment is swung by the second motor 42 in a range of, for example, +355 degrees when any of the horizontal directions is defined as 0 degrees.

In the above description, each actuator is a motor, but the motor is not limited to the actuator, and may be a hydraulic cylinder, a pneumatic cylinder, or the like.

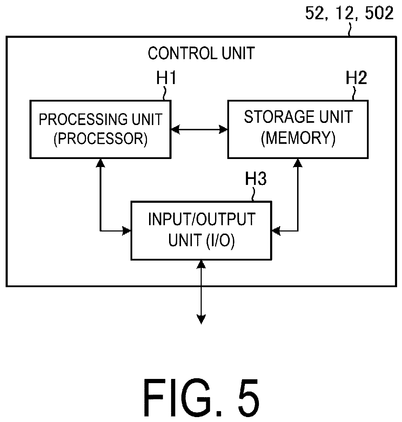

FIG. 5 is a diagram illustrating an example of a hardware configuration of the control unit 52 of the controller 5, the control unit 12 of the terminal 10, or the control unit 502 of the controller 500. In FIG. 5, the control unit 52, 12, 502 has a configuration of a general computer, and includes a processing unit (processor) H1, a storage unit (memory) H2, and an input/output unit (I/O) H3. The storage unit H2 includes a read only memory (ROM), a random access memory (RAM), a nonvolatile-RAM (NV-RAM), a solid state drive (SSD), and a hard disk drive (HDD).

The processing unit H1 executes processing based on data stored in the storage unit H2 or data input from the input/output unit H3 in accordance with a program stored in the storage unit H2. Data obtained by the processing is output from the input/output unit H3.

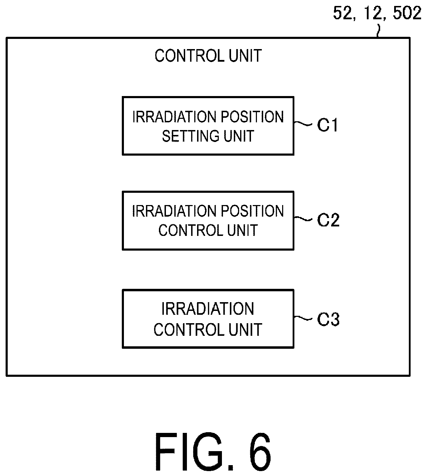

FIG. 6 is a diagram illustrating an example of a functional configuration the control unit 52 of the controller 5, the control unit 12 of the terminal 10, or the control unit 502 of the controller 500. In FIG. 6, the control unit 52, 12, 502 includes an irradiation position setting unit C1, an irradiation position control unit C2, and an irradiation control unit C3.

The irradiation position setting unit C1 has a function of setting a position or a range (a range is designated by a plurality of positions or the like) to be irradiated with ultraviolet rays for sterilization. The irradiation position control unit C2 has functions of controlling the vertical angle adjustment unit 3 and the horizontal angle adjustment unit 4 of the sterilizer 1 based on the setting information and controlling the position irradiated with ultraviolet rays. The irradiation control unit C3 has a function of turning ON and OFF the ultraviolet rays, and of changing the intensity of the ultraviolet rays (the intensity of the ultraviolet rays may or may not be changeable depending on the light sources), or changing the wavelengths of the ultraviolet rays (switching can be performed between a wavelength settable for human body irradiation and a wavelength not settable for human body irradiation but has a high sterilization effect) based on the setting information, the detection signals of the first sensor unit 61 and the second sensor unit 62.

### Real Time Operation by User

In FIGS. 1 to 6, the user operates the terminals 10 and the like to change the status of the sterilizer 1 (1A, 1B), the irradiation status of the light source unit 21, and the orientation status of the light source unit 21. For example, when the user operates the terminal 10 to turn ON the light source unit 21, the controller 5 turns ON the light source unit 21 via the light source unit drive circuit 53, based on the irradiation control signal corresponding to the operation information corresponding to the turn ON. With the light source unit 21 turned ON, the sterilizer 1 irradiates the sterilization target space S with the ultraviolet rays UV in a radiating form, to sterilize the air in the sterilization target space S and an object in the sterilization target space S with the ultraviolet rays UV. When the user operates the terminal 10 to change the irradiation range of the ultraviolet rays UV in the sterilization target space S in the vertical direction, the controller 5 drives the first motor 32 via the first motor drive circuit 54 based on the drive control signal for the first motor 32 corresponding to the operation information corresponding to the adjustment of the vertical angle to rotate the light source unit 21 in the vertical direction. When the user operates the terminal 10 to change the irradiation range of the ultraviolet rays UV in the sterilization target space S in the horizontal direction, the controller 5 drives the second motor 42 via the second motor drive circuit 55 based on the drive control signal for the second motor 42 corresponding to the operation information corresponding to the adjustment of the horizontal angle to rotate the light source unit 21 in the horizontal direction. Thus, the user who wants to sterilize an object in the sterilization target space S rotates the light source unit 21 in the vertical direction and the horizontal direction, to move the irradiation range of the ultraviolet rays UV from the light source unit 21 to the object, and performs the sterilization.

Also with a real-time operation by the user, automatic irradiation stop described below may be performed (ultraviolet ray irradiation from the light source unit 21 is stopped when the second sensor unit 62 detects proximity of an object in a direction, and in this direction, the first sensor unit 61 has detected an object having a body temperature, or when the second sensor unit 62 detects proximity of an object).

### Automatic Irradiation based on Set Position (Fixed Irradiation Time)

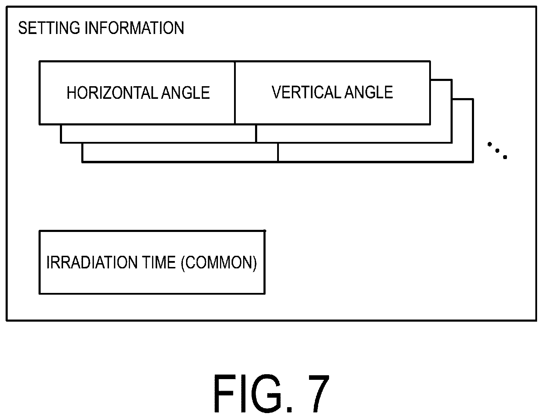

FIG. 7 is a diagram illustrating an example of a data structure of setting information held in the control unit 52 of the controller 5, the control unit 12 of the terminal 10, or the control unit 502 of the controller 500. In FIG. 7, the setting information includes a plurality of sets of a horizontal angle and a vertical angle. A set of a horizontal angle and a vertical angle indicates an irradiation direction corresponding to one position. The horizontal angle is an angle (pan) to be controlled by the horizontal angle adjustment unit 4 (FIG. 1). The vertical angle is an angle (tilt) to be controlled by the vertical angle adjustment unit 3 (FIG. 1). The setting information includes an irradiation time common to the positions.

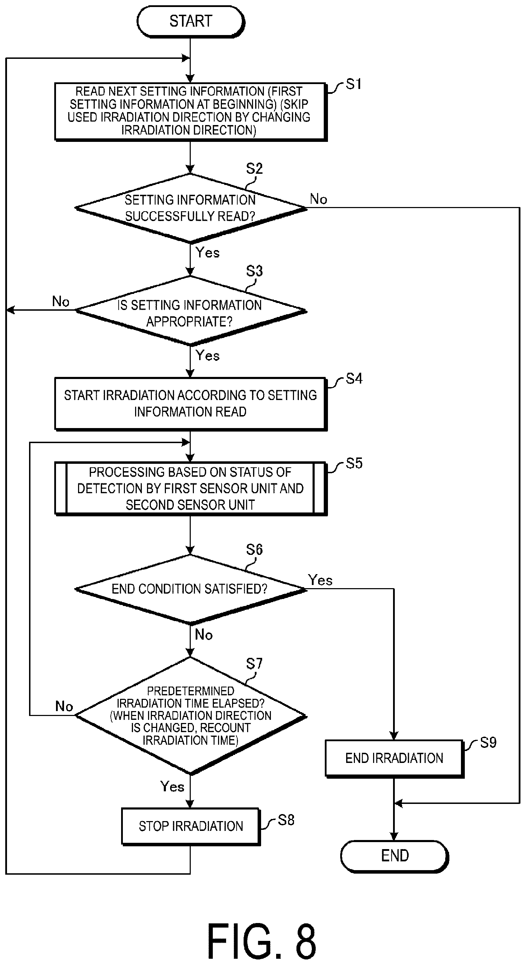

FIG. 8 is a flowchart illustrating an example of control by the control unit 52 of the controller 5, the control unit 12 of the terminal 10, or the control unit 502 of the controller 500. In FIG. 8, when the processing starts, the control unit 52 or the like reads the next setting information (first setting information at the beginning) (step S 1). Note that an irradiation direction of irradiation having been completed is skipped through a change in irradiation direction described below. An irradiation direction of irradiation not completed due to the change in irradiation direction may be regarded as unprocessed to be read again.

Next, the control unit 52 or the like determines whether the setting information was found and has been successfully read (step S2). When the setting information was not found or when the unprocessed irradiation direction was determined to have failed to be read (No in step S2), the control unit 52 or the like ends the processing.

When the setting information was determined to be successfully read (Yes in step S2), the control unit 52 or the like determines whether the setting information is appropriate by determining whether the setting information is not contradictive (step S3). When the setting information read was determined not to be appropriate (No in step S3), the control unit 52 or the like proceeds to reading of the next setting information (step S1).

When the setting information read was determined to be appropriate (Yes in step S3), the control unit 52 or the like starts the ultraviolet ray irradiation based on the setting information read (step S4). Specifically, the ultraviolet ray irradiation is performed with the optical axis of the light source unit 21 set to be at the horizontal angle and the vertical angle included in the setting information (FIG. 7).

Next, the control unit 52 or the like executes processing according to the status of detection by the first sensor unit 61 and the second sensor unit 62 (step S5). This processing will be described in detail below.

Next, the control unit 52 or the like determines whether an end condition is satisfied with an end instruction from the user or the like (step S6), and when the end condition was determined not to be satisfied (No in step S6), then determines whether common irradiation time included in the setting information (FIG. 7) has elapsed (step S7). When the common irradiation time was determined not to have elapsed yet (No in step S7), the control unit 52 or the like proceeds to the processing according to the status of detection by the first sensor unit 61 and the second sensor unit 62 (step S5). When the irradiation direction has been changed in the middle of the processing, the irradiation time is counted again from the beginning.

When the common irradiation time was determined to have elapsed (Yes in step S7), the control unit 52 or the like stops the ultraviolet ray irradiation (step S8), and proceeds to the reading of the next setting information (step S 1).

When the end condition was determined to have been satisfied (Yes in step S6) in the determination (step S6) for the end condition, the control unit 52 or the like stops the ultraviolet ray irradiation (step S9), and ends the processing.

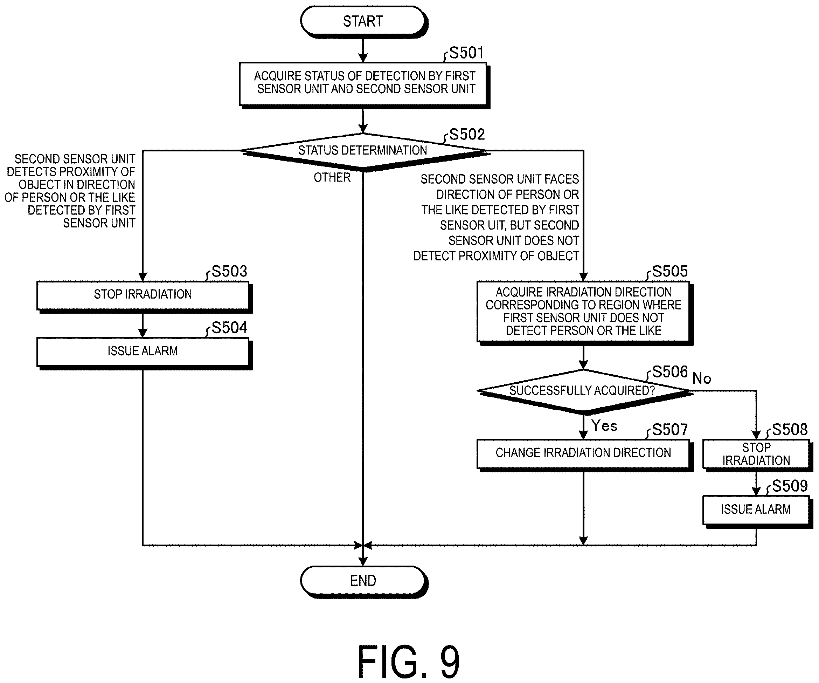

FIG. 9 is a flowchart illustrating an example of the processing in step S5 in FIG. 8. In FIG. 9, when the processing starts, the control unit 52 or the like acquires the status of detection by the first sensor unit 61 and the second sensor unit 62 (step S501) and performs status determination (step S502).

Here, the second sensor unit 62 has detected proximity (proximity within 30 cm for example) of an object in a direction of a person or the like detected by the first sensor unit 61. In this status in the status determination (step S502), the control unit 52 or the like stops the ultraviolet ray irradiation (step S503). In an environment where an object other than an object having a body temperature such as a person or the like will not be at a position close to the second sensor unit 62 (such as a case where furniture and fixtures are not arranged at a position close to the second sensor unit 62), when the second sensor unit 62 detects the proximity of the object regardless of the detection status of the first sensor unit 61, the proximity of the object having a body temperature such as a person or the like may be determined and the ultraviolet ray irradiation may be stopped (step S503).

Next, the control unit 52 or the like issues an alarm (step S504) and ends the processing. The alarm notifies a user such as a manager of stoppage of the sterilization operation using ultraviolet ray irradiation.

When a person or the like enters the proximity of, that is, within 30 cm from the light source unit 21 of the sterilizer 1, the ultraviolet ray irradiation is immediately stopped, so that damage such as cell destruction otherwise occurring can be prevented in advance, whereby the safety can be guaranteed.

On the other hand, in a status in the status determination (Step S502), the second sensor unit 62 faces the direction of a person or the like detected by the first sensor unit 61 but the second sensor unit 62 does not detect (detects no) proximity of an object. In this status, the control unit 52 or the like acquires an irradiation direction corresponding to a region where the first sensor unit 61 has not detected a person or the like (step S505). The irradiation direction corresponding to a region where a person or the like has not been detected is a direction toward the center of a region of a certain range where a person or the like is not detected.

Upon acquiring the irradiation direction corresponding to the region where the first sensor unit 61 has not detected a person or the like (Yes in Step S506), the control unit 52 or the like changes the irradiation direction of the ultraviolet rays to the acquired direction while the ultraviolet rays are being emitted (step S507), and ends the processing.

Even if a person or the like is present in the ultraviolet ray irradiation direction, there is no problem in terms of safety as long as the distance is not too short to require immediate irradiation stop. Thus, by changing the irradiation direction to a direction without a person or the like not being present while continuing the irradiation, the sterilization operation can be continued while guaranteeing safety.

When the irradiation direction corresponding to the region where the first sensor unit 61 has not detected a person or the like fails to be acquired (No in Step S506), the control unit 52 or the like stops the ultraviolet ray irradiation (step S508), issues an alarm (step S509), and ends the processing.

On the other hand, the status in the status determination results in other status (step S502), the control unit 52 or the like ends the processing.

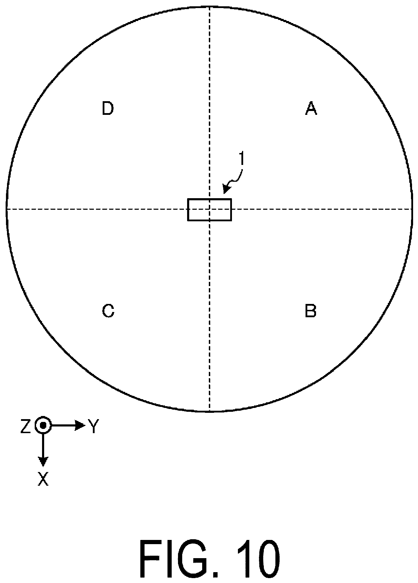

FIG. 10 is a plan view illustrating an example of an area being an ultraviolet ray irradiation target. In FIG. 10, a circular region is divided into four fan-shaped areas A to D, and the sterilizer 1 is assumed to be installed in a ceiling portion at the center.

In FIG. 10, for example, when a person or the like enters the area A and suddenly comes close to the sterilizer 1 while the area A is being irradiated with ultraviolet rays, the ultraviolet ray irradiation is immediately stopped and an alarm is issued (steps S503 and S504 in FIG. 9).

On the other hand, when a person or the like enters the area A being irradiated with the ultraviolet rays but does not come close to the sterilizer 1, the irradiation direction is changed to, for example, the area C isolated from the area A and including no person or the like (steps S505 to S507 in FIG. 9). If a person or the like is present also in the area C, there is no destination target, and to this destination target, the irradiation direction is to be changed. Thus, the irradiation of ultraviolet rays is stopped, and an alarm is issued (steps S508 and S509 in FIG. 9). Although the area B and the area D including no person or the like and adjacent to the area A are not set as the irradiation direction change destination in consideration of the influence on the person or the like in the area A, the irradiation direction of the ultraviolet ray may be changed to the area B or the area D including no person or the like if the person or the like in the area A would not be affected.

In addition, when the distance to the existing person or the like can be acquired by a distance sensor or the like at the time of selecting the irradiation direction change destination, the irradiation direction can be selected to be separated from the existing person or the like by a distance as long as possible.

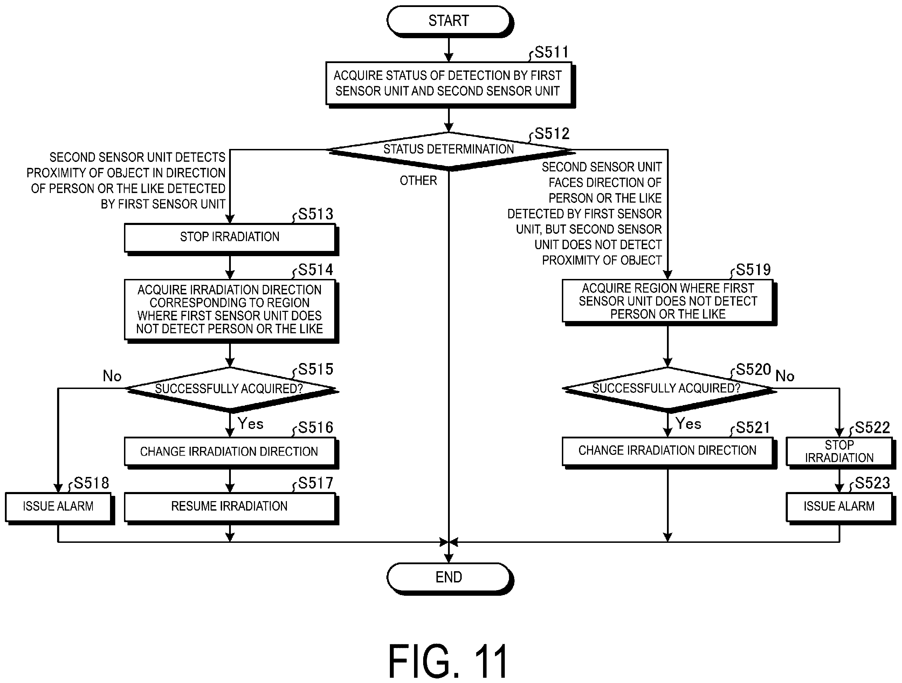

FIG. 11 is a flowchart illustrating another example of the processing in step S5 in FIG. 8. In FIG. 11, when the processing starts, the control unit 52 or the like acquires the status of detection by the first sensor unit 61 and the second sensor unit 62 (step S511) and performs status determination (step S512).

Here, in a status in the status determination (step S512), the second sensor unit 62 has detected proximity (proximity within 30 cm for example) of an object in a direction, and in this direction, the first sensor unit 61 has detected a person or the like is determined. In this status, the control unit 52 or the like stops the ultraviolet ray irradiation (step S513). In an environment where an object other than an object having a body temperature such as a person or the like will not be at a position close to the second sensor unit 62, when the second sensor unit 62 detects the proximity of the object regardless of the detection status of the first sensor unit 61, the proximity of the object having a body temperature such as a person or the like may be determined and the ultraviolet ray irradiation may be stopped (step S513).

Then, the control unit 52 or the like acquires an irradiation direction corresponding to the region where the first sensor unit 61 has not detected a person or the like (step S514). Then, upon acquiring the irradiation direction corresponding to the region where the first sensor unit 61 has not detected a person or the like (Yes in Step S515), the control unit 52 or the like changes the irradiation direction of the ultraviolet rays to the acquired direction (step S516), resumes the ultraviolet ray irradiation (step S517), and ends the processing.

While the ultraviolet ray irradiation is stopped when the proximity of a person or the like is detected for the sake of safety, in other regions including no person or the like, resumption of the ultraviolet ray irradiation would not be a problem, and thus the sterilization operation can be resumed and continued.

When the irradiation direction corresponding to the region where the first sensor unit 61 has not detected a person or the like fails to be acquired (No in Step S515), the control unit 52 or the like issues an alarm (step S518), and ends the processing.

On the other hand, in a state in the status determination (step S512), the second sensor unit 62 faces the direction of a person or the like detected by the first sensor unit 61 but the second sensor unit 62 does not detect (detects no) proximity of an object. In this status, the control unit 52 or the like acquires an irradiation direction corresponding to a region where the first sensor unit 61 has not detected a person or the like (step S519). Upon acquiring the irradiation direction corresponding to the region where the first sensor unit 61 has not detected a person or the like (Yes in Step S520), the control unit 52 or the like changes the irradiation direction of the ultraviolet rays to the acquired direction while the ultraviolet rays are being emitted (step S521), and ends the processing.

When the irradiation direction corresponding to the region where the first sensor unit 61 has not detected a person or the like fails to be acquired (No in Step S520), the control unit 52 or the like stops the ultraviolet ray irradiation (step S522), issues an alarm (step S523), and ends the processing.

On the other hand, the status determination results in other (step S512), the control unit 52 or the like ends the processing.

While the operation example based on the setting information in FIG. 7 has been described, the setting information includes other various patterns. For example, when the irradiation time is set together with the horizontal angle and the vertical angle, the ultraviolet ray irradiation is performed based on the irradiation time set for each irradiation direction.

When the irradiation intensity of the ultraviolet rays can be changed and the irradiation intensity is set together with the horizontal angle and the vertical angle, the ultraviolet ray irradiation is performed based on the irradiation intensity set for each irradiation direction (with constant irradiation time). When the irradiation intensity of the ultraviolet rays can be changed and the irradiation time and the irradiation intensity are set together with the horizontal angle and the vertical angle, the ultraviolet ray irradiation is performed based on the irradiation time and the irradiation intensity set for each irradiation direction.

When the wavelength of the ultraviolet rays can be changed and the wavelength is set together with the horizontal angle and the vertical angle as well as the irradiation time and the irradiation intensity, the ultraviolet ray irradiation is performed based on the wavelength and the like.

In addition, when the distance to the sterilization target can be acquired by a distance sensor or the like, and when the distance is set together with the horizontal angle and the vertical angle as well as the irradiation time, the irradiation intensity, and the wavelength, the ultraviolet ray irradiation is performed based on the irradiation time, the irradiation intensity, and the wavelength corresponding to the set distance.

Furthermore, when a range defined by a plurality of irradiation directions is set instead of the individual irradiation direction based on the horizontal angle and the vertical angle, the set range is irradiated with the ultraviolet rays. Also in this case, irradiation time, irradiation intensity, wavelength, distance, and the like can be combined.

The embodiment of the present invention has been described above, but the present invention is not limited to the embodiment described above, and various modifications are possible without departing from the spirit of the present invention.

As described above, a sterilization system according to an embodiment includes: a light source unit configured to emit ultraviolet rays in a radiating form; a horizontal angle adjustment unit configured to adjust a horizontal angle of the light source unit by causing rotation of the light source unit in a horizontal direction and/or a vertical angle adjustment unit configured to adjust a vertical angle of the light source unit by causing rotation of the light source unit in a vertical direction; a first sensor unit configured to detect an object having a body temperature; a second sensor unit configured to detect proximity of an object; and a controller configured to read setting information including the horizontal angle and/or the vertical angle, control the rotation caused by the horizontal angle adjustment unit and/or the vertical angle adjustment unit, and control irradiation of the ultraviolet rays by the light source unit based on a status of the detection by the first sensor unit and the second sensor unit. Thus, the safety for a human body and the like can be improved.

The first sensor unit is a pyroelectric infrared sensor, and the second sensor unit is a proximity sensor. Thus, the presence of a human body or the like can be appropriately recognized.

The first sensor unit is disposed at a fixed position to overlook an entire range to be irradiated with the ultraviolet rays, and the second sensor unit is disposed at a movable position to be oriented in an irradiation direction of the ultraviolet rays together with the light source unit. Thus, the presence of a human body or the like can be appropriately recognized.

When the second sensor unit detects proximity of an object in a direction of an object having a body temperature and detected by the first sensor unit or when the second sensor unit detects proximity of an object, the controller stops the irradiation of the ultraviolet rays by the light source unit. Thus, the safety for a human body and the like can be improved.

After stopping the irradiation of the ultraviolet rays, the controller acquires the direction corresponding to the region where the object having a body temperature has not been detected by the first sensor unit, controls the horizontal angle adjustment unit and/or the vertical angle adjustment unit to cause rotation to the direction, and resumes the irradiation of the ultraviolet rays by the light source unit. Thus, the sterilization operation can continue while guaranteeing safety.

When the second sensor unit does not detect proximity of an object in a direction of an object having a body temperature and detected by the first sensor unit, the controller acquires a direction corresponding to a region where an object having a body temperature has not been detected by the first sensor unit, and controls the horizontal angle adjustment unit and/or the vertical angle adjustment unit to cause rotation to the direction to change the irradiation direction of the ultraviolet rays from the light source unit. Thus, the sterilization operation can continue while guaranteeing safety.

Moreover, the present invention is not limited to the embodiment described above. A configuration obtained by appropriately combining the above-mentioned constituent elements is also included in the present invention. Further effects and modified examples can be easily derived by a person skilled in the art. Thus, a wide range of aspects of the present invention is not limited to the embodiment described above and may be modified variously.

### Reference Signs List

1, 1A, 1B Sterilizer, 21 Light source unit, 3 Vertical angle adjustment unit, 4 Horizontal angle adjustment unit, 5 Controller, 51 Communication unit, 52 Control unit, 53 Light source unit drive circuit, 54 First motor drive circuit, 55 Second motor drive circuit, 56 Sensor unit interface circuit, 10 Terminal, 500 Controller, C1 Irradiation position setting unit, C2 Irradiation position control unit, C3 Irradiation control unit

## Claims

1. A sterilization system comprising:
a light source unit configured to emit ultraviolet rays in a radiating form;
a horizontal angle adjustment unit configured to adjust a horizontal angle of the light source unit by causing rotation of the light source unit in a horizontal direction; and/or
a vertical angle adjustment unit configured to adjust a vertical angle of the light source unit by causing rotation of the light source unit in a vertical direction;
a first sensor unit configured to detect an object having a body temperature;
a second sensor unit configured to detect proximity of an object; and
a controller configured to read setting information including the horizontal angle and/or the vertical angle, control the rotation caused by the horizontal angle adjustment unit and/or the vertical angle adjustment unit, and control irradiation of the ultraviolet rays by the light source unit based on a status of detection by the first sensor unit and the second sensor unit.

2. The sterilization system according to claim 1, wherein
the first sensor unit is a pyroelectric infrared sensor, and
the second sensor unit is a proximity sensor.

3. The sterilization system according to claim 1 or 2, wherein
the first sensor unit is disposed at a fixed position to overlook an entire range configured to be irradiated with the ultraviolet rays, and
the second sensor unit is disposed at a movable position to be oriented in an irradiation direction of the ultraviolet rays together with the light source unit.

4. The sterilization system according to any one of claims 1 to 3, wherein
when the second sensor unit detects proximity of an object in a direction of an object having a body temperature and detected by the first sensor unit or when the second sensor unit detects proximity of an object, the controller stops the irradiation of the ultraviolet rays from the light source unit.

5. The sterilization system according to claim 4, wherein
after stopping the irradiation of the ultraviolet rays, the controller acquires the direction corresponding to the region where the object having a body temperature is not detected by the first sensor unit, controls the horizontal angle adjustment unit and/or the vertical angle adjustment unit to cause rotation to the direction, and resumes the irradiation of the ultraviolet rays from the light source unit.

6. The sterilization system according to any one of claims 1 to 5, wherein
when the second sensor unit does not detect proximity of an object in a direction of an object having a body temperature and detected by the first sensor unit, the controller acquires a direction corresponding to a region where an object having a body temperature is not detected by the first sensor unit, and controls the horizontal angle adjustment unit and/or the vertical angle adjustment unit to cause rotation to the direction to change the irradiation direction of the ultraviolet rays from the light source unit.

7. A sterilizer comprising:
a light source unit configured to emit ultraviolet rays in a radiating form;
a horizontal angle adjustment unit configured to adjust a horizontal angle of the light source unit by causing rotation of the light source unit in a horizontal direction; and/or
a vertical angle adjustment unit configured to adjust a vertical angle of the light source unit by causing rotation of the light source unit in a vertical direction;
a first sensor unit configured to detect an object having a body temperature;
a second sensor unit configured to detect proximity of an object; and
a controller configured to read setting information including the horizontal angle and/or the vertical angle, control the rotation caused by the horizontal angle adjustment unit and/or the vertical angle adjustment unit, and control irradiation of the ultraviolet rays by the light source unit with reference to a status of detection by the first sensor unit and the second sensor unit.

8. A controller configured to perform for
a light source unit configured to emit ultraviolet rays in a radiating form,
a horizontal angle adjustment unit configured to adjust a horizontal angle of the light source unit by causing rotation of the light source unit in a horizontal direction, and/or
a vertical angle adjustment unit configured to adjust a vertical angle of the light source unit by causing rotation of the light source unit in a vertical direction,
a first sensor unit configured to detect an object having a body temperature, and
a second sensor unit configured to detect proximity of an object,
reading of setting information including the horizontal angle and/or the vertical angle, controlling of the rotation caused by the horizontal angle adjustment unit and/or the vertical angle adjustment unit, and controlling of irradiation of the ultraviolet rays by the light source unit with reference to a status of detection by the first sensor unit and the second sensor unit.

9. A control method comprising, by a computer, executing for
a light source unit configured to emit ultraviolet rays in a radiating form,
a horizontal angle adjustment unit configured to adjust a horizontal angle of the light source unit by causing rotation of the light source unit in a horizontal direction, and/or
a vertical angle adjustment unit configured to adjust a vertical angle of the light source unit by causing rotation of the light source unit in a vertical direction,
a first sensor unit configured to detect an object having a body temperature, and
a second sensor unit configured to detect proximity of an object,
reading of setting information including the horizontal angle and/or the vertical angle, controlling of the rotation caused by the horizontal angle adjustment unit and/or the vertical angle adjustment unit, and controlling of irradiation of the ultraviolet rays by the light source unit with reference to a status of detection by the first sensor unit and the second sensor unit.

10. A control program causing a computer to execute for
a light source unit configured to emit ultraviolet rays in a radiating form,
a horizontal angle adjustment unit configured to adjust a horizontal angle of the light source unit by causing rotation of the light source unit in a horizontal direction, and/or
a vertical angle adjustment unit configured to adjust a vertical angle of the light source unit by causing rotation of the light source unit in a vertical direction,
a first sensor unit configured to detect an object having a body temperature, and
a second sensor unit configured to detect proximity of an object,
reading of setting information including the horizontal angle and/or the vertical angle, controlling of the rotation caused by the horizontal angle adjustment unit and/or the vertical angle adjustment unit, and controlling of irradiation of the ultraviolet rays by the light source unit with reference to a status of detection by the first sensor unit and the second sensor unit.
